# EUROPÄISCHE PATENTANMELDUNG

(11) **EP 2 034 007 A2**
(43) Veröffentlichungstag der Anmeldung: **11.03.2009**
(21) Anmeldenummer: 08163179.8
(22) Anmeldetag: 28.08.2008
(51) Int. Cl.: C12M 1/02, C12M 1/08

(54) **Mittel zum Heizen eines liegenden, rohrförmigen Fermenters**

(30) Priorität: 04.09.2007 CH 13792007
(71) Anmelder: Kompogas AG, 8152 Glattbrugg (CH)
(72) Erfinder: Kern, Daniel, 8302, Kloten (CH)
(74) Vertreter: Schneider Feldmann AG Patent- und Markenanwälte

(57) **Zusammenfassung**

In einem Fermenter (1) sind mehrere Heizlanzen (6), welche die Fermenterwand (2) durchsetzen, exzentrisch ausserhalb des Bereiches der Antriebswelle (3) des Rührwerkes angeordnet. Diese Heizlanzen (6) ruhen auf einer balkonartigen Platte (7). Die Heizlanze (6) ist über eine Vorlaufleitung (10) verbunden. Durch diese strömt ein Heizmedium in die Heizlanze (6). Die Heizlanze ist aus zwei konzentrischen Rohren gebildet. Das Heizmedium strömt zwischen einem äusseren Rohr (60) und einem inneren Rohr (62) nach unten und steigt über das innere Rohr (62) nach oben bis zu einer Rücklaufleitung (11) um sicherzustellen dass die Heizlanze durch den anliegenden Druck des Pfropfstromes (P) keinen Schaden nimmt, ruht die Heizlanze (6) auf einer balkonartigen Platte (7). Bevorzugterweise ist die Heizlanze (6) austauschbar angeordnet. Entsprechend ist auf die Platte (7) ein Lagerrohr (13) geschweisst in das die Heizlanze (6) eintaucht.

Auf diese Weise wird in einem pfropfstrombetriebenen Fermenter ein einfaches Mittel zum Heizen realisiert, welches sich preiswert realisieren lässt, den Pfropfstrom kaum beeinflusst und zudem auswechselbar gestaltet sein kann, so dass die Montage und Demontage besonders einfach ist, was sowohl die Herstellung als auch den Betrieb des Fermenters sicherer und kostengünstiger macht.

## Beschreibung

Die vorliegende Erfindung betrifft ein Mittel zum Heizen eines liegenden, trog- oder rohrförmigen Fermenters der im Pfropfstromverfahren betrieben wird und einem in Längsrichtung diesen zentrisch durchsetzendes Rührwerk mit einer Antriebswelle und mehreren voneinander distanziert auf der Welle angeordnete Rührarme aufweist.

Fermenter sind chemische Reaktionsbehälter in denen Fermentationsprozesse durchgeführt werden. In der Mehrheit aller Fälle sind diese Fermenter mit Flüssigkeiten gefüllt und zur Steuerung des Fermentationsverfahrens mit Rührwerken und Mittel zum Heizen ausgerüstet. Üblich ist es dabei, dass in den Fermentern Flüssigkeiten vorhanden sind die chargenweise eingetragen werden, das Fermentationsverfahren durchgeführt wird und danach der Fermenter vollständig entleert wird. Um solche Fermenter bzw. deren Inhalt zu beheizen ist es durchaus üblich Mittel zum Heizen vorzusehen, die im Wesentlichen einem Tauchsieder ähnlich sind.

Dies zeigt beispielsweise die GB-2 144 767 A. Hierbei handelt es sich um einen liegenden, rohrförmigen Fermenter in dem mehrere wendelförmige Heizrohre von oben nach unten den Fermenter durchsetzen und ein zentrisches Saugrohr umgeben. Über dieses Saugrohr wird der flüssige, zu fermentierende Stoff von unten angesaugt im Bereich des Heizrohres aufgewärmt und oben über Düsen wieder abgegeben. Der Betrieb eines solchen Fermentes zur Erzeugung von Methangas wird chargenweise betrieben.

In ähnlicher Weise wird auch in stehenden Behältern das zu fermentierende Gut beheizt, wie dies beispielsweise aus der JP 2005198618 hervorgeht. Eine ungewöhnliche Lösung zeigt die DE-102004027077-A. Hier ist ein stehender Fermenter gezeigt dessen Rührwerk mit einer Hohlwelle versehen ist, welches beliebig im Fermenterbehälter einseitig drehbar lagert und auf deren Welle ein oder mehrere heizbare Rührblätter angebracht sind. Dieses System eignet sich ebenfalls nur für einen stehenden, ruhenden Fermenter in dem das zu fermentierende Gut ebenfalls chargenweise ein- und ausgetragen wird. Typisch für solche Systeme sind Fermenter für Agrarbetriebe bei denen mittels des Fermenters aus flüssigem Dung Methangas gewonnen wird.

Bei liegenden, rohrförmigen Fermenter die im Pfropfstromverfahren betrieben werden und bei denen der zu fermentierende Inhalt einen sehr geringen Flüssiganteil enthält, sind die Probleme völlig anders. Bei liegenden, rohrförmigen Fermenter, die im Pfropfstrombetrieb arbeiten, wirken auf die Heizelemente, wenn diese in den Fermenterraum hineinragen, extrem hohe Kräfte. Da gleichzeitig auch noch eine zentrische Welle den Fermenter üblicherweise durchsetzt, sind solche in den Fermenter hineinragende Heizelemente nicht einfach anbringbar. Aus diesem Grund hat der Anmelder bis heute die Fermenter mit einem aussen um den Fermenter angebrachten Heizmantel versehen. Während sich dies bei Betonfermenter mit einem sinnvollen Aufwand realisieren lässt, ist dies bei Stahlfermenter ausgesprochen aufwendig. Entsprechend wurden preiswertere Ausführungsvarianten gesucht.

So wurde aus der US 2004/0,224,399 A ein liegender Fermenter bekannt, auf dem auf der Innenseite oben ein zentrales Zuführungssystem angebracht ist und von dem aus vertikal freihängend lanzenförmige Heizmittel in das zu fermentierende Gut hineinragen. Da bei diesem Fermenter kein zentrales Rührwerk den Fermenter durchsetzt, sondern im Bodenbereich eine Schnecke angebracht ist die sowohl einen gewissen Mischvorgang als auch eine Förderung realisiert, können die Heizlanzen relativ weit in das zu fermentierende Gut eintauchen. Da sich sowohl die auftretenden Kräfte durch die Durchmischung als auch durch die Förderung praktisch auf den oberen Bereich begrenzen, sind die Kräfte, die durch das geförderte, sich zu fermentierende Gut auftreten, nur gering und somit dürften vermutlich die Heizlanzen diesen Kräften standhalten. Bei liegenden pfropfstrombetriebenen Fermenter, die aber mit einer zentralen Rührwelle durchsetzt sind, lässt sich diese Lösung nicht realisieren. Die auftretenden Kräfte führen zu Verformungen der Heizlanzen und Anwendungen dieser Art haben beim Anmelder zu Undichtigkeiten der Heizlanzen geführt.

Entsprechend hat man auch nach anderen Mitteln zur Heizung im pfropfstrombetriebenen Rohrfermenter gesucht. Die EP-1 524 315 A zeigt eine solche weitere Möglichkeit. Hier sind diese Heizmittel praktisch als in Längsrichtung des Fermenters verlaufende Tauchsieder realisiert, die an der Rohrwelle befestigt sind und mit dieser rotieren. Eine solche Lösung ist relativ aufwendig und bei grossen Rohrfermenter, die durchaus eine Länge von bis zu 50 m aufweisen, kann eine solche Lösung praktisch nur in den Endbereichen der Fermenter eingesetzt werden, so dass sich eine Wärmesteuerung über die gesamte Länge des Fermenters hinweg vermutlich so nicht realisieren lässt.

Es ist daher die Aufgabe der vorliegenden Erfindung, Mittel zum Heizen eines liegenden, rohrförmigen Fermenters, der im Pfropfstromverfahren betrieben wird, gemäss dem Oberbegriff des Patentanspruches 1 zu schaffen, die den Kräften in einem solchen Fermenter der mit hohem Feststoffanteil betrieben wird, Stand zu halten vermögen.

Diese Aufgabe löst ein Mittel zum Heizen eines liegenden, rohrförmigen Fermenters mit den Merkmalen des Patentanspruches 1.

Weitere vorteilhafte Ausgestaltungsformen des Erfindungsgegenstandes gehen aus den abhängigen Patentansprüchen hervor, deren Bedeutung und vorteilhafte Wirkungsweise in der nachfolgenden Beschreibung unter Bezug der anliegenden Zeichnung erläutert sind.

In der Zeichnung ist eine bevorzugte Ausführungsform des Erfindungsgegenstandes dargestellt und nachfolgend beschrieben. Es zeigt:
- Fig. 1: einen vereinfachten, vertikalen Querschnitt durch einen liegenden, rohrförmigen Fermenter mit Rührwerk;
- Fig. 2: einen vertikalen Teillängsschnitt durch einen Fermenter sowie durch die in diesem Bereich angebrachte Heizlanze mit Verkürzung in vertikaler Richtung.

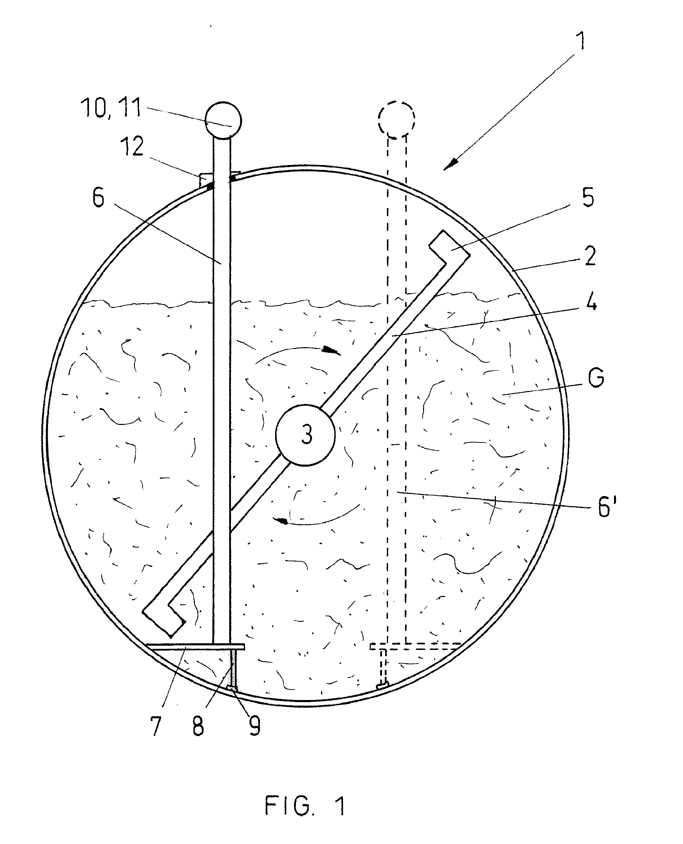

In der Figur 1 ist der Fermenter insgesamt mit 1 bezeichnet. Dieser ist senkrecht zu seiner Längsausdehnung geschnitten, entsprechend bildet bei einem rohrförmigen Fermenter die Fermenterwand 2 einen Kreis. Für die Erfindung ist jedoch ein zylindrischer bzw. rohrförmiger Fermenter nicht zwingend. In Frage kommt insbesondere auch ein liegender, druckförmiger Fermenter der im Prinzip in einem entsprechenden Vertikalschnitt U-förmig mit gerundetem Boden und einem vorzugsweise flachen Deckel ausgebildet sein kann. Der Fermenter wird im Pfropfstromverfahren betrieben. In der Zeichnung gemäss der Figur 1 ist somit die Pfropfstrom-Förderrichtung P senkrecht zur Zeichnungsebene. Das im Fermenter zu fermentierende Gut, meist Grüngut, ist mit G bezeichnet. Dieses wird eingangsseitig meist mittels Schneckenförderer unter Druck eingegeben und schiebt damit das bereits darin liegende Gut zur Ausgangsseite. Mittels eines Rührwerks wird dieses Gut G vermischt. Es bewegt sich somit im Fermenter in dessen Längsrichtung schraubenförmig von der Eingangsseite zur Ausgangsseite. Diese Förderungsart wird als Pfropfstrom bezeichnet.

Mittig in Längsrichtung wird der Fermenter durch das Rührwerk durchsetzt, welches eine Antriebswelle 3 umfasst, an dem in regelmässigen Abständen Rührarme 4 angeordnet sind. Diese Rührarme 4 können endseitig ein Scharblech 5 aufweisen.

Der hier gezeigte Fermenter ist selbstverständlich lediglich vereinfacht dargestellt. So ist der meist den Fermenter umgebende Isolationsmantel, die Lagerung des Fermenters deren Steuerungen und Zuleitungen die meist darin angebrachten Sensoren zur Überwachung der Betriebsparameter sowie insbesondere auch die Entnahmemittel zum Absaugen des darin entstandenen Methangases nicht dargestellt, da diese Elemente für die hier zu beschreibende Erfindung nicht von Bedeutung sind.

Das erfindungsgemässe Mittel zum Heizen eines solchen Fermenters besteht im Wesentlichen aus mindestens einer, bevorzugterweise jedoch aus einer Vielzahl von Heizlanzen 6. Eine solche Heizlanze ist äusserlich lediglich als Rohr zu erkennen. Dieses kann im Querschnitt im Prinzip eine beliebige Form aufweisen, jedoch wird bevorzugterweise die Heizlanze aus zylindrischen Rohren gebildet. Die zylindrische Form wird nicht zuletzt deshalb bevorzugt, da so der Strömungswiderstand relativ gering ist.

Die erfindungsgemässen Heizlanzen 6 sind bezüglich der Längsachse des Fermenters exzentrisch ausserhalb des Bereiches der Antriebswelle 3 angeordnet. Im oberen Bereich des Fermenters 1 ist die Heizlanze 6 durch eine dichtende Durchführung 12 gehalten. Sinnvollerweise wird man die Heizlanzen so anbringen, dass diese auswechselbar im Fermenter 1 gehalten sind. Dies erlaubt die Herausnahme für Kontroll- und Reperaturzwecke. Im unteren Bereich ist die Heizlanze 6 auf einer Platte 7 ruhend gelagert. Diese Platte 7 ist praktisch balkonartig an der Fermenterwand 2 angeformt. Diese Ausgestaltung hat den Vorteil, dass der Strömungsquerschnitt hierdurch kaum verringert wird. Dies ist von Bedeutung, da sich das fermentierende Gut G sich so kaum rückstauend aufbauen kann. Entsprechend ist die Platte 7 so verlaufend, dass diese exakt in Pfropfstromrichtung P verläuft. Zur Erhöhung der Stabilität kann die balkonartig angeformte Platte 7 mit Stützen 8 versehen sein, die mit Füssen 9 an der Fermenterwand 2 angeschweisst sind.

Die einzelnen Heizlanzen 6 sind immer zwischen zwei benachbarten Rührarmen 4, genügend von denselben distanziert, angeordnet. Bei der Anordnung einer Vielzahl solcher Heizlanzen 6 wird man bevorzugterweise abwechslungsweise diese einmal auf der einen und einmal auf der anderen Seite der Antriebswelle 3 anordnen. Dies ist in der Zeichnung durch eine zweite, strichliniert gezeichnete Heizlanze 6' angedeutet. Hier ist die Heizlanze 6 in Blickrichtung von einem Rührarm 4 dargestellt, während die folgende Heizlanze 6' in Blickrichtung hinter denselben Rührarm 4 angeordnet ist. Folglich sind hier die Heizlanzen alternativ einmal links, einmal rechts von der Antriebswelle 3 angeordnet. Dies ist zwar nicht zwingend, jedoch eine sinnvolle Anordnung.

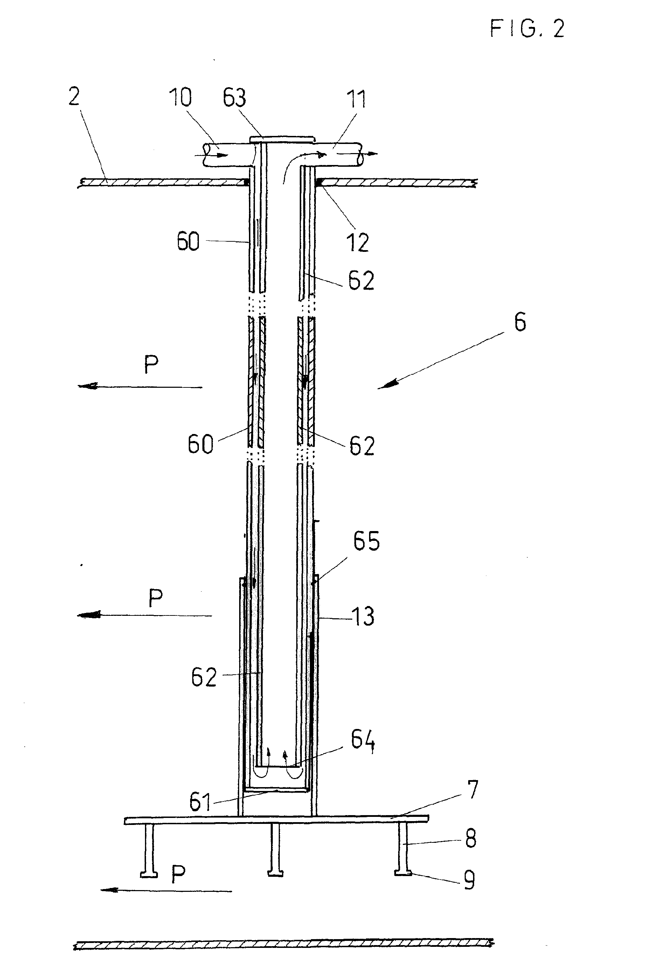

Bezüglich des Aufbaus der Heizlanze wird nun mit Bezug auf die Figur 2 dieselbe im Detail erläutert. Die Heizlanze 6 hat ein äusseres Rohr 60, welches am unteren Ende mit einer Blindplatte 61 verschlossen ist. Konzentrisch darin verläuft ein inneres Rohr 62. Dieses innere Rohr 62 ist am unteren Ende bei 64 offen. Das innere Rohr 62 endet ein Stück weit oberhalb der Blindplatte 61. Am oberen Ende ist das innere Rohr 62 mit einem Deckel 63 verschlossen. Im Prinzip könnte die Platte 7 auf der die Heizlanze 6 ruht auch gleichzeitig die Blindplatte 61 bilden. Dies hat jedoch den Nachteil, dass dann das äussere Rohr der Heizlanze 6 nicht auswechselbar ist.

Ausserhalb der Fermenterwand 2 erkennt man die Vorlaufleitung 10. Diese mündet in das äussere Rohr 60. Das warme Heizmedium, welches über die Vorlaufheizung 10 einströmt, gelangt somit in das äussere Rohr 60 und strömt in diesem bzw. im Zwischenraum zwischen dem äussern Rohr 60 und dem inneren Rohr 62 nach unten. Dieser konzentrische Innenraum zwischen dem äusseren Rohr 60 und dem inneren Rohr 62 bildet somit innerhalb der Heizlanze 6 den Vorlaufbereich. Dieser erstreckt sich bis zur Blindplatte 61 und von dort steigt das Heizwasser bei 64 im Innenraum des inneren Rohres 62 nach oben. Das innere Rohr bildet folglich den Rücklauf innerhalb der Heizlanze 6. Das innere Rohr 62 kommuniziert dann mit einer Rücklaufleitung 11 von wo aus das Wasser entweder in eine Wanddurchlaufheizung gelangt oder aber sozusagen wiederum zur Vorlaufleitung für eine nachfolgende Heizlanze wird.

Die Heizlanze die am unteren Ende gelagert ist kann im Prinzip auf verschiedenen Arten auf der Platte 7 ruhen. In der hier dargestellten, bevorzugten Ausführungsform ist auf der Platte 7 ein Lagerrohr 13 aufgeschweisst. Dieses Lagerrohr 13 ist durch einen auf die Platte 7 aufgeschweissten Rohrabschnitt gebildet. Dieser Rohrabschnitt bildet einen Lagertopf. In diesem Lagertopf lagert die Heizlanze 6 mit ihrem unteren Ende. Im Prinzip kann die Blindplatte 61 dabei auf die Platte 7 zu liegen kommen, doch ist dies für eine stabile Lagerung nicht erforderlich. Der innere Durchmesser des Lagerrohres 13 ist geringfügig grösser als der Aussendurchmesser des äusseren Rohres 60 der Heizlanze 6. Im oberen Endbereich des Lagerrohres 13 kann eine Dichtung, beispielsweise eine O-Ring-Dichtung 65, angebracht sein. Diese Dichtung dient dazu, dass die in geringem Masse immer auch vorhandene Feuchtigkeit sich im Zwischenraum zwischen dem Lagerrohr 13 und dem äusseren Rohr 60 nicht absetzen kann und damit sichergestellt wird, dass hier nicht eine Schicht entsteht die ein Auswechseln der Heizlanze 6 extrem erschwert. Dieser Zwischenraum kann aber auch mit einem entsprechenden Fett oder anderem Mittel das plastisch oder elastisch bleibt abgedichtet werden.

### Bezugszeichenliste:

- 1: Fermenter
- 2: Fermenterwand
- 3: Antriebswelle
- 4: Rührarm
- 5: Scharblech
- 6: Heizlanze
- 6': Heizlanze
- 7: Platte
- 8: Stütze
- 9: Fuss
- 10: Vorlaufleitung
- 11: Rücklaufleitung
- 12: dichtende Durchführung
- 13: Lagerrohr
- 60: äusseres Rohr der Heizlanze
- 61: Blindplatte
- 62: inneres Rohr
- 63: Deckel
- 64: offenes Ende von 62
- G: Grüngut
- P: Pfropfstromrichtung
- D: Drehrichtung Rührwerk

## Patentansprüche

1. Mittel zum Heizen eines liegenden, trag- oder rohrförmigen Fermenters (1) der im Pfropfstromverfahren betrieben wird und einem in Längsrichtung diesem zentrisch durchsetzendes Rührwerk mit einer Antriebswelle (3) und mehrere voneinander distanziert auf der Antriebswelle (3) angeordnete Rührarme (4) aufweist, **dadurch gekennzeichnet, dass** das Mittel zum Heizen aus mindestens einer Heizlanze (6) besteht, die den liegenden Fermenter (1) exzentrisch zur Längsachse ausserhalb des Bereiches der Antriebswelle (3) und zwischen zwei benachbarten Rührarmen (4) angeordnet ist, wobei die Heizlanze (6) im oberen seitlichen Bereich des liegenden Fermenters (1) die Fermenterwand (2) dichtend durchsetzt und im unteren seitlichen Bereich des Fermenters (1) auf einer am Fermenter (1) befestigten Platte (7) befestigt ruht.

2. Mittel nach Anspruch 1, **dadurch gekennzeichnet, dass** die Heizlanze (6) aus zwei konzentrischen Rohren (60, 62) gebildet ist und der Vorlauf zwischen einem äusseren (60) und einem inneren (62) der beiden Rohre (60, 62) verläuft, während der Rücklauf im inneren des Innenrohres verläuft und wobei das äussere Rohr (60) mit einem Deckel (63) verschlossen ist.

3. Mittel nach Anspruch 1, **dadurch gekennzeichnet, dass** die Platte (7) auf der die Heizlanze (6) im unteren Bereich des Fermenters (1) ruht parallel zur Strömungsrichtung verlaufend balkonartig angeordnet ist.

4. Mittel nach Anspruch 3, **dadurch gekennzeichnet, dass** die balkonartige Platte (7) mit mindestens einer auf der Unterseite angeordneten Stütze (8) verstärkt ist.

5. Mittel nach Anspruch 4, **dadurch gekennzeichnet, dass** die balkonartige Platte (7) mehrere auf der Unterseite der Platte (7) in Strömungsrichtung hintereinander angeordnete Stützen (8) aufweist.

6. Mittel nach Anspruch 3, **dadurch gekennzeichnet, dass** auf der Oberseite der Platte (7) ein offenes Lagerrohr (13) aufgeschweisst ist, in dem die Heizlanze (6) mit seinem freien Ende eintaucht.

7. Mittel nach Anspruch 6, **dadurch gekennzeichnet, dass** die Heizlanze (6) im oberen Bereich des Fermenters (1) gedichtet lose hindurchgeführt ist und auswechselbar im Lagerrohr (13) ruht.
